(19) Europäisches Patentamt
European Patent Office
Office européen des brevets

(11) **EP 2 856 935 A1**

(12) **EUROPÄISCHE PATENTANMELDUNG**

(43) Veröffentlichungstag:
08.04.2015 Patentblatt 2015/15

(51) Int Cl.:
*A61B 5/00* (2006.01)  *A61B 5/06* (2006.01)
*A61B 18/12* (2006.01)

(21) Anmeldenummer: **14180599.4**

(22) Anmeldetag: **12.08.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **26.09.2013 US 201361882649 P**

(71) Anmelder: **Biotronik AG**
**8180 Bülach (CH)**

(72) Erfinder: **Christian, Moß**
**10589 Berlin (DE)**

(74) Vertreter: **Lindner-Vogt, Karin L. et al**
**Biotronik SE & Co. KG**
**Corporate Intellectual Properties**
**Woermannkehre 1**
**12359 Berlin (DE)**

(54) **Medizinische Vorrichtung zur Auswertung eines Temperatursignals**

(57)　Medizinische Vorrichtung (10; 10'; 10"; 10''') mit einer Auswerteeinheit (12) und mit einer Elektrodenleitung (14; 14'; 14"). Die Elektrodenleitung (14; 14'; 14") umfasst wenigstens einen Temperatursensor (20; 20'; 20"). Der Temperatursensor (20; 20'; 20") ist ausgebildet ein Temperatursignal (63; 63a; 63b; 63c; 63d; 63e; 63f) an die Auswerteeinheit (12) zu liefern. Die Auswerteeinheit (12) ist ausgebildet periodische Schwankungen ei- nes Signalpegels des Temperatursignals (63; 63a; 63b; 63c; 63d; 63e; 63f) auszuwerten und ein eine Wandständigkeit der Elektrodenleitung (14; 14'; 14") qualifizierendes Auswertungsausgangsignal in Abhängigkeit davon zu erzeugen, ob periodische Schwankungen eines Signalpegels des Temperatursignals unterhalb oder oberhalb eines vorbestimmten Grenzwertes liegen.

FIG. 5

**Beschreibung**

Die Erfindung betrifft eine medizinische Vorrichtung zur Auswertung eines Temperatursignals mit einer Auswerteeinheit und mit einer einen Temperatursensor umfassenden Elektrodenleitung und ein Verfahren zur Auswertung eines Temperatursignals unter Verwendung der medizinischen Vorrichtung.

Katheter werden heutzutage in medizinischen Anwendungen verwendet um innerhalb des Körpers liegende Regionen bzw. Hohlräume eines Patienten zu erreichen. Katheter sind biegeschlaffe Schläuche, die in Abhängigkeit ihres Aufbaus mannigfaltige Funktionen ausführen können. Beispielsweise kann ein Katheter mit Elektroden ausgestattet sein um Energie auf um die Elektroden liegendes Gewebe zu übertragen. Katheter mit Elektroden werden beispielsweise für die renale Denervation verwendet, bei der selektiv afferente sympathische Nervenfasern (des Sympathischen Nervensystems) in den Nierenarterien durch die Abgabe von Wärmeenergie an die Nervenfasern durchtrennt bzw. verödet werden. Die renale Denervation wird zur Therapie von therapieresistenter arterieller Hypertonie verwendet, welche als Bluthochdruck definiert wird, der trotz medikamentöser Therapie mit blutdrucksenkenden Medikamenten, darunter wenigstens ein Diuretikum, nicht im angestrebten Blutdruckzielbereich liegt.

Die Kontrolle über die genaue Energiemenge, die von den Elektroden an das Gewebe abgegeben wird, wird dadurch erschwert, dass die Lage des Katheters am Einsatzort nicht genau kontrolliert werden kann. Hierdurch ist der Katheter u. U. nicht im direkten Kontakt mit dem Gewebe an das die Energie abgegeben werden soll und ein Teil der Energie wird anstatt dessen an das Blut abgegeben. Dies kann zur Koagulation bzw. Verklumpung des Blutes führen, was im Hinblick auf eine sichere Therapie unbedingt vermieden werden muss. Das verklumpte Blut kann in diesem Fall durch den Verschluss von Adern Schäden an den Organen bewirken und im schlimmsten Fall auch Gehirn- oder Herzgefäße verschließen, was zum Tode des Patienten führen kann. Daher ist ein Wandkontakt zwischen Elektroden und Gewebe essentiell für eine erfolgreiche Durchführung der renalen Denervations-Prozedur. Eine Strategie zum Vermeiden von Schäden ist es die Energieabgabe schrittweise zu erhöhen, um einen langsameren Temperaturanstieg zu erzeugen, der eine geringere Gefahr birgt das Blut zu verklumpen.

Zur Bestimmung des Wandkontaktes wird im Allgemeinen der Temperaturanstieg über die Zeit oder die Impedanz des Gewebes gemessen, da die Impedanz mit längerer Dauer der Energieabgabe an das Gewebe abnimmt. Tierversuche haben jedoch gezeigt, dass durch Auswerten der Impedanz alleine keine Aussage über einen zuverlässigen Wandkontakt getroffen werden kann. Das Fehlen eines Wandkontaktes lässt sich beispielsweise durch einen geringen oder nicht vorhandenen Temperaturanstieg nach Energieabgabe messen.

Die Auswertung des Temperaturanstiegs ist jedoch zeitaufwendig, was ein Nachteil bei der Verwendung als Abschaltkriterium ist.

EP 0 566 725 B1 zeigt eine Ablationselektrode für Ablationskatheter mit thermisch isolierten thermischen Sensoren. Der Ablationskatheter hat einen Energie emittierenden mit einer Energiequelle verbundenen Körper zum Kontaktieren mit Gewebe, der Temperatursensoren enthält, die auf dem Körper angeordnet und von diesem thermisch isoliert sind. Die Temperatursensoren messen die Temperatur des Gewebes und sind mit einem Hochfrequenzgenerator verbunden, der die Energie für den Energie emittierenden Körper erzeugt. Die Energieabgabe des Hochfrequenzgenerators kann entsprechend der gemessenen Temperatur an den Temperatursensoren oder einer gemessenen Impedanz des Gewebes angepasst werden, um eine Temperatur des Gewebes in bestimmten Temperaturbereichen bzw. Impedanzbereichen zu erhalten.

In EP 2 338 430 B1 wird eine medizinische Sonde mit Dehnungsmessfühler präsentiert. Die Sonde umfasst einen flexiblen Einbringungsschlauch, dessen distales Ende ausgebildet ist in Kontakt mit Gewebe eines Körperhohlraums gebracht zu werden und eine Sensorröhre, die ein elastisches Material im distalen Ende des flexiblen Einbringungsschlauch enthält, das ausgebildet ist sich als Antwort auf vom Gewebe auf das distale Ende wirkende Kräfte zu verformen. Eine Vielzahl von Dehnungsmessfühlern ist an verschiedenen Stellen auf der Oberfläche der Sensorröhre aufgebracht, die ein jeweiliges Signal als Antwort auf Verformungen der Sensorröhre erzeugen. Die Sonde enthält des Weiteren wenigstens einen temperaturkompensierenden Dehnungsmessfühler, der ausgebildet ist Signale zu generieren um Temperaturänderungen in der Vielzahl von Dehnungsmessfühlern auszugleichen.

EP 1 827 277 B1 zeigt einen Katheter mit einer Mehrzahl von mikrofabrizierten Temperatursensoren. Die Temperatursensoren sind in einer Umgebung einer Spitze eines distalen Endes des Katheters auf einer äußeren Oberfläche angeordnet. Die Spitze wird von einer Elektrode gebildet. Die Temperatursensoren enthalten eine dünne Sensorschicht, die durch Temperaturänderung ihren Widerstand ändern kann. Des Weiteren kann ein Temperatursensor den Umfang des Katheters umschließen.

Ziel der Erfindung ist es eine verbesserte medizinische Vorrichtung und ein verbessertes Verfahren zu entwickeln, um eine Wandständigkeit einer Elektrodenleitung bzw. einen Kontakt zwischen einer Wand und einer Elektrodenleitung zu erfassen.

Erfindungsgemäß wird dies erreicht durch eine medizinische Vorrichtung mit einer Auswerteeinheit und mit einer Elektrodenleitung, die wenigstens einen Temperatursensor umfasst. Der Temperatursensor ist ausgebildet ein Temperatursignal an die Auswerteeinheit zu liefern. Die Auswerteeinheit ist ausgebildet, periodische Schwankungen eines Signalpegels des Temperatursignals auszuwerten und ein eine Wandständigkeit der Elektrodenleitung qualifizierendes Auswertungsausgangsignal in Abhängigkeit davon zu erzeugen, ob periodische Schwankungen eines Signalpegels des Temperatursignals unterhalb oder oberhalb eines vorbestimmten Grenzwertes liegen.

Die Erfinder haben erkannt, dass periodische Schwankungen eines Signalpegels eines Temperatursignals ein schnelles und somit auch vorteilhaftes Kriterium zur Bestimmung einer Wandständigkeit liefern. Dies erlaubt es Energie einzusparen, da unnötige Energieabgabe, falls die Elektrodenleitung nicht in Kontakt mit der Wand ist, an ein eine Wand umgebendes Fluid verringert werden kann. Hierbei kann das Kriterium genutzt werden, um schneller zu entscheiden, wann die Energieabgabe zu unterbrechen ist. Die Elektrodenleitung kann dann neu ausgerichtet werden, um einen Wandkontakt herzustellen. Das Kriterium kann beispielsweise auch zur Unterbrechung der Energieabgabe im Fall eines schlechten Wandkontaktes bei einer renalen Denervation genutzt werden. Hierbei kann schneller erkannt werden, ob ein guter Wandkontakt beispielweise einer bipolaren Elektrodenleitung, bzw. eines bipolaren Katheters mit der Wand besteht, indem kein Frequenzanteil der Herzfrequenz im Spektrum der Temperatur vorhanden ist. Eine schnellere Unterbrechung der Energieabgabe kann erfolgen, falls das Spektrum einen Frequenzanteil der Herzfrequenz aufweist. Die schnellere Unterbrechung der Energieabgabe bei schlechtem Wandkontakt verringert die Blutgerinnung und dadurch ausgelöste Ausbildung von Blutklumpen, die zu Verstopfungen in den Blutgefäßen und Schäden im Körper des Patienten führen können.

Bevorzugt ist die Elektrodenleitung biegeschlaff, wodurch sie in innere kurvenaufweisende Hohlräume geschoben werden kann, beispielsweise in Gefäße oder Organe eines Körpers, künstliche Organe eines Testsystems oder eines Wirkstoffgewinnungssystems oder in andere Gefäße oder Fluidleitungen. Die Elektrodenleitung kann ein Katheter sein. Dieser kann eine Beschichtung haben oder ein unbeschichteter Katheter sein. In einer bevorzugten Ausgestaltung umfasst die Elektrodenleitung einen biegeschlaffen Schlauch. Die Elektrodenleitung kann gekühlt sein, beispielsweise kann ein Kühlmittel im Inneren des biegeschlaffen Schlauchs der Elektrodenleitung geführt sein. In einer weiteren Ausgestaltung kann die Elektrodenleitung ein Ablationskatheter mit Ablationselektrode sein. Gemäß einer besonders bevorzugten Variante ist der Katheter ein spezieller Katheter für die renale Denervation. Die Elektrodenleitung kann auch einen expandierbaren Ballon, beispielsweise an ihrem distalen Ende, enthalten oder ein Ballonkatheter sein.

Der oder die Temperatursensoren sind bevorzugt in der Nähe der Elektrode oder Elektroden angeordnet. Insbesondere bevorzugt sind der Temperatursensor oder die Temperatursensoren in der Nähe einer Ablationselektrode angeordnet. Es können auch mehrere Temperatursensoren in der Nähe einer jeweiligen Ablationselektrode angeordnet sein. Es ist auch denkbar, dass die Elektrodenleitung einen den Umfang der Elektrodenleitung umspannenden Temperatursensor enthält. Bevorzugt sind die Temperatursensoren von den Elektroden thermisch isoliert. Insbesondere bevorzugt sind die Temperatursensoren auf einer äußeren Oberfläche der Elektrodenleitung angeordnet. Die Temperatursensoren können beispielsweise Thermoelemente, Sensorschichten, die bei Temperaturänderung ihren elektrischen Widerstand ändern, oder dergleichen sein.

In einer bevorzugten Ausgestaltung ist die Elektrodenleitung der medizinischen Vorrichtung eine bipolare Elektrodenleitung, beispielsweise ein bipolarer Katheter oder dergleichen, mit einem Ablationselektrodenpaar für eine bipolare Ablation. Bevorzugt sind die beiden Ablationselektroden dabei zueinander in einer geringen Entfernung angeordnet, die dafür geeignet ist, eine Ablation mit Hilfe beider Ablationselektroden durchzuführen, indem diese einen Stromkreis über eine Wand schließen. Die Auswerteeinheit erzeugt im Fall einer bipolaren Elektrodenleitung ein eine Wandständigkeit der bipolaren Elektrodenleitung anzeigendes Auswertungsausgangsignal, wenn periodische Schwankungen eines Signalpegels des Temperatursignals unterhalb des vorbestimmten Grenzwertes liegen. Zusätzlich oder alternativ kann die Auswerteeinheit auch ein keine Wandständigkeit der bipolaren Elektrodenleitung anzeigendes Auswertungsausgangsignal erzeugen, wenn periodische Schwankungen eines Signalpegels des Temperatursignals oberhalb des vorbestimmten Grenzwertes liegen. Beispielmessungen haben gezeigt, dass das Nicht-Erreichen einer Zieltemperatur von 70° C zu einem Maximalwert von 0,6° C bis 1,0° C für periodische Schwankungen eines Signalpegels des Temperatursignals im Temperaturspektrum führt, wenn keine Wandständigkeit der bipolaren Elektrodenleitung vorliegt und zu einem Maximalwert von 0,08° C, wenn eine Wandständigkeit der bipolaren Elektrodenleitung vorliegt. Hieraus kann ein vorbestimmter Grenzwert ermittelt werden. Der vorbestimmte Grenzwert entspricht bevorzugt einem Wert zwischen 0,1° C und 1,0° C, besonders bevorzugt einem Wert zwischen 0,2° C und 0,8° C und insbesondere bevorzugt einem Wert zwischen 0,3° C und 0,6° C.

In einer alternativen Ausgestaltung ist die Elektrodenleitung der medizinischen Vorrichtung eine unipolare Elektrodenleitung, beispielsweise ein unipolarer Katheter mit Gegenelektrode bzw. Neutralelektrode oder dergleichen, mit einer Ablationselektrode für eine unipolare Ablation. Bevorzugt ist die eine Ablationselektrode dabei mit einer außerhalb des Wand enthaltenden Systems, beispielsweise an einem Körper, befestigten Neutralelektrode im elektrischen Kontakt, die dafür geeignet ist, eine Ablation mit Hilfe der Ablationselektrode durchzuführen, indem diese einen Stromkreis durch eine Wand und beispielsweise den Körper mit der Ablationselektrode schließt. Die Auswerteeinheit erzeugt im Fall einer unipolaren Elektrodenleitung ein eine Wandständigkeit der unipolaren Elektrodenleitung anzeigendes Auswertungsausgangsignal, wenn periodische Schwankungen eines Signalpegels des Temperatursignals oberhalb des vorbestimmten Grenzwertes liegen. Zusätzlich oder alternativ kann die Auswerteeinheit auch ein keine Wandständigkeit der unipolaren Elektrodenleitung anzeigendes Auswertungsausgangsignal erzeugen, wenn periodische Schwankungen eines Signalpegels des Temperatursignals unterhalb des vorbestimmten Grenzwertes liegen. Beispielmessungen haben gezeigt, dass das Nicht-Erreichen einer Zieltemperatur von 70° C zu einem Maximalwert von 0,1° C für periodische Schwankungen eines Signalpegels des Temperatursignals im Temperaturspektrum führt, wenn keine Wandständigkeit der Elektrodenleitung vorliegt, während eine Wandständigkeit für Maximalwerte oberhalb von 0,2° C gefunden werden kann. Hieraus kann ein vorbestimmter Grenzwert ermittelt werden. Der vorbestimmte Grenzwert entspricht bevorzugt einem Wert zwischen 0,05° C und 0,6° C, besonders bevorzugt einem Wert zwischen 0,1° C und 0,4° C und insbesondere bevorzugt einem Wert zwischen 0,2° C und 0,3° C.

In einer bevorzugten Ausgestaltung enthält die medizinische Vorrichtung eine Energiequelle. Die Energiequelle ist bevorzugt mit der Elektrodenleitung elektrisch verbunden und kann der Elektrodenleitung Energie zuführen. Die Energiequelle kann auch mit der Auswerteeinheit elektrisch verbunden sein. Die Auswerteeinheit kann in diesem Fall ein Energiequellenabschaltsignal erzeugen, wenn die Auswerteeinheit ein keine Wandständigkeit der Elektrodenleitung anzeigendes Auswertungsausgangsignal erzeugt oder erzeugt hat. Die Auswerteeinheit kann zum Abschalten der Energiequelle das Energiequellenabschaltsignal an die Energiequelle oder eine Steuereinheit der Energiequelle übermitteln. Im Betrieb der Energiequelle kann das Energiequellenabschaltsignal die Energieabgabe von der Energiequelle an die Elektrodenleitung, beispielsweise für eine Ablation, abschalten oder unterbrechen.

In einer Ausgestaltung kann eine Elektrodenleitung für eine renale Denervation, beispielsweise in einer Renalarterie einer Niere oder einer künstlichen Renalarterie eines künstlichen Testsystems genutzt werden. Im Falle einer renalen Denervation mit einer bipolaren Elektrodenleitung, beispielsweise einem bipolaren Katheter, erfolgt die Abschaltung der Energiequelle bei nicht ausreichendem Wandkontakt basierend auf der Detektion einer Pulsfrequenz im Temperatursignal bei der Energieabgabe. Nach dem Starten der Energieabgabe wird fortlaufend eine Zeit-Frequenz-Transformation der Temperaturdaten berechnet. Die Temperaturdaten werden dafür aus dem analogen Temperatursignal des Temperatursensors gewonnen, indem dieses zuerst in einem Temperatursignalverstärker, beispielsweise mit integrierter Vergleichsstellenkompensation, verstärkt und dann in einem Analog-zu-Digital-Wandler digitalisiert wird. Gegebenenfalls wird das digitalisierte Temperatursignal noch in einer Mikrokontrollereinheit kodiert und einer Computereinheit zur Auswertung bereitgestellt. Alternativ kann auch das digitalisierte Temperatursignal in einem Ablationsgenerator oder der Auswerteeinheit ausgewertet werden. Im Falle einer Energieabgabe bei der renalen Denervation erwärmt sich das um die Elektroden liegende Gewebe der Renalarterie, wenn ein Wandkontakt bzw. eine Wandständigkeit besteht. In diesem Fall dient die Erwärmung zur Verödung von sympathischen Nervenfasern und der Blutstrom durch die Renalarterie hat nur einen geringen Einfluss auf die Wärmeentwicklung bzw. den Temperaturverlauf. Daher ist bei einer Zeit-Frequenz-Transformation der Temperaturdaten keine Pulsfrequenz im Spektrum sichtbar. Wenn der Katheter einen schlechten Wandkontakt bzw. keine Wandständigkeit hat, wird der Temperaturverlauf durch den Blutfluss (Volumenstrom) $F = \dfrac{dV}{dt}$, mit V dem Blutvolumen und t der Zeit beeinflusst. Durch die Aktivität des Herzens unterliegt der Blutfluss einer ständigen Änderung im Herzrhythmus. Dies führt bei schlechtem Wandkontakt zu einer Kühlung, die als Pulsfrequenz im Temperaturspektrum sichtbar wird.

Die renale Denervation kann auch mit einer unipolaren Elektrodenleitung durchgeführt werden. Bei der Verwendung einer unipolaren Elektrodenleitung, beispielsweise eines unipolaren Katheters kehrt sich der Effekt um. Über eine Hochfrequenz-Zuführung von der Energiequelle wird Energie an die Elektrodenleitung zur Energieabgabe zugeführt. Außerhalb des Körpers ist eine Neutralelektrode (Klebeelektrode) am Körper befestigt. Im Gegensatz zum bipolaren Katheter ist bei schlechtem Wandkontakt die Pulsfrequenz im Temperaturspektrum nicht sichtbar. Bei gutem Wandkontakt zeigt sich jedoch eine schwache Amplitude der Pulsfrequenz im Temperaturspektrum des unipolaren Katheters.

Generell ist es denkbar, dass Messprinzip für die Erkennung der Wandständigkeit bei der renalen Denervation auch vor Therapiebeginn zu nutzen, um die Qualität des Wandkontaktes zu bestimmen. Beispielsweise kann mit einem bipolaren Katheter für einen kurzen Zeitraum eine geringe Leistung abgegeben werden, die nur zu einer unwesentlichen Erwärmung der Umgebung des Katheters führt. Basierend auf einer Auswertung der Temperaturdaten kann damit auf die Qualität des Wandkontaktes geschlossen werden.

Bevorzugt wird die Auswertung der periodischen Schwankungen eines Signalpegels des Temperatursignals mit Hilfe einer Zeit-Frequenz-Transformation des Temperatursignals ermöglicht. Die Zeit-Frequenz-Transformation kann beispielsweise eine Fourier-Transformation sein, die unter Verwendung beispielsweise eines FFT (Fast-Fourier-Transformation), Wavelet-Transformation, Short-Time-Fourier-Transformation Algorithmus' oder dergleichen, durchgeführt wird.

Der Frequenzbereich des Zeit-Frequenz-transformierten Temperatursignals kann beispielsweise zwischen 0 Hz (0 bpm (beats per minute)) und 20 Hz (1200 bpm), bevorzugt zwischen 0,2 Hz (12 bpm) und 4,2 Hz (252 bpm), besonders bevorzugt zwischen 0,8 Hz (48 bpm) und 3,4 Hz (204 bpm) und insbesondere bevorzugt zwischen 1,1 Hz (66 bpm) und 2,0 Hz (120 bpm) ausgewertet werden.

In einer Ausgestaltung umfasst das Verfahren zur Auswertung eines Temperatursignals unter Verwendung der medizinischen Vorrichtung ein zur Verfügung stellen eines Temperatursignals und ein Auswerten der periodischen Schwankungen eines Signalpegels des Temperatursignals. Das Temperatursignal muss nicht von einem Temperatursensor geliefert werden und kann beispielsweise auch künstlich erzeugt sein. Bevorzugt wird bei der Ausgestaltung des Verfahrens ein eine Wandständigkeit der Elektrodenleitung qualifizierendes Auswertungsausgangsignal in Abhängigkeit davon erzeugt, ob periodische Schwankungen eines Signalpegels des Temperatursignals unterhalb oder oberhalb eines vorbestimmten Grenzwertes liegen. Im Mittel können beispielsweise wenigstens 3,3 Temperaturmesswerte pro Sekunde, besonders bevorzugt wenigstens 6,6 Temperaturmesswerte pro Sekunde und insbesondere bevorzugt wenigstens 22 Temperaturmesswerte pro Sekunde ausgewertet werden.

Zusätzlich kann das Verfahren einen Schritt umfassen, der eine Zeit-Frequenz-Transformation des Temperatursignals durchführt. Hierfür kann beispielsweise ein analoges Signal zuerst digitalisiert werden und dieses digitalisierte Signal dann über eine Zeit-Frequenz-Transformation von der Zeitdomäne in den Frequenzbereich transformiert werden. Bevorzugt werden im Mittel wenigstens z. B. 3,3 Temperaturmesswerte pro Sekunde (entsprechend einer Samplingrate (Abtastfrequenz) von 3,3 Hz), besonders bevorzugt wenigstens 6,6 Temperaturmesswerte pro Sekunde (entsprechend einer Samplingrate von 6,6 Hz) und insbesondere bevorzugt wenigstens 22 Temperaturmesswerte pro Sekunde (entsprechend einer Samplingrate von 22 Hz) genutzt um die Zeit-Frequenz-Transformation des Temperatursignals durchzuführen. Die Auswertung des Zeit-Frequenz-transformierten Signals kann dann beispielsweise über die Auswertung der Amplituden von bevorzugten Frequenzbereichen erfolgen.

Die Abtastfrequenz $f_s$ zum Digitalisieren der Temperaturdaten ist bevorzugt mindestens doppelt so groß wie die maximal im analogen Temperatursignal auftretende Frequenz. Wird angenommen, dass die maximale Frequenz durch eine Pulsfrequenz eines Menschen vorgegeben ist, so müssen beispielsweise Temperatursignale bis zu 3,333 Hz (200 bpm) erfasst werden. Die Abtastfrequenz beträgt also bevorzugt wenigstens 6,667 Hz. Um Aliasing-Effekte zu vermeiden wird $f_s$ bevorzugt mit einer Frequenz von wenigstens 10 Hz, besonders bevorzugt 22 Hz und insbesondere bevorzugt mit einer Frequenz von wenigstens 100 Hz verwendet. Die digitalisierten Daten werden mit einem Algorithmus zur Zeit-Frequenz-Transformation, beispielsweise FFT (Fast-Fourier-Transformation) in den Frequenzbereich transformiert. Je kleiner die Frequenzauflösung dabei sein soll, desto größer muss die Anzahl N der Punkte gewählt werden, die in die Berechnung mit einfließen.

Die Anzahl N der Punkte ergibt sich mit $N = \dfrac{f_s}{\Delta f}$ , wobei $\Delta f$ die Auflösung im Frequenzbereich angibt. Beispielsweise werden für eine Auflösung der Pulsfrequenz von 0,167 Hz (10 bpm) somit $N = \dfrac{22\,Hz}{10/60s} = 132$ benötigt.

Der Algorithmus zur Berechnung der FFT kann nur auf Datenlängen operieren, die einer Zweierpotenz entsprechen, daher wird eine Datenlänge bzw. Anzahl N von 128 oder 256 benötigt, die beispielsweise durch Kürzen der Datenlänge oder Ergänzen mit Nullen erzeugt werden kann. Die Zeit T für die Erfassung von N Datenwerten

beträgt $T = N \cdot \dfrac{1}{f_s}$ . Bei einer Abtastrate von 22 Hz ist für eine Pulsfrequenzauflösung von 0,167 Hz (10 bpm) eine Aufzeichnung über 6s notwendig. Ein Vorteil ist, dass das Verfahren eine schnelle Detektion der Pulsfrequenz in den Daten ermöglicht, wodurch ein sicheres Abschalten der Energieabgabe sichergestellt ist. In praktischen Versuchen hat sich gezeigt, dass innerhalb von 3 s nach Beginn der Energieabgabe bestimmt werden kann, ob ein Anteil der Pulsfrequenz in den Daten vorhanden ist.

In einer weiteren Ausgestaltung des Verfahrens enthält das Verfahren als ersten Schritt ein Starten einer Energieabgabe, beispielsweise der Energiequelle an die Elektrodenleitung. Bevorzugt enthält das Verfahren einen Schritt, der ein Beenden der Energieabgabe, beispielsweise von der Energiequelle an die Elektrodenleitung beinhaltet, wenn die Auswerteeinheit ein keine Wandständigkeit der Elektrodenleitung anzeigendes Auswertungsausgangsignal erzeugt bzw. erzeugt hat.

In einer bevorzugten Ausgestaltung enthält die medizinische Vorrichtung eine eine gepulste Fluidströmung erzeugende Pulsgebereinheit. Die Pulsgebereinheit kann beispielsweise den Hohlraum eines künstlichen Organs oder dergleichen mit einem Fluid versorgen und eine Pulsfrequenz für die Fluidströmung erzeugen. Die Pulsgebereinheit kann auch ausgebildet sein einen variierenden Puls zu erzeugen, beispielsweise mit Hilfe einer randomisierten Prozedur in einem vorbestimmten Frequenzbereich.

Die Erfindung soll nun anhand von in den Figuren schematisch abgebildeten Ausführungsbeispielen näher erläutert werden. Von den Figuren zeigen:

Fig. 1      zeigt ein erstes Ausführungsbeispiel einer medizinischen Vorrichtung mit einer Elektrodenleitung in einer Renalarterie einer Niere;

Fig. 2　eine schematische Darstellung eines Ausführungsbeispiels einer medizinischen Vorrichtung mit einer bipolaren Elektrodenleitung;

Fig. 3　eine schematische Darstellung eines Ausführungsbeispiels einer medizinischen Vorrichtung mit einer unipolaren Elektrodenleitung;

Fig. 4　eine schematische Darstellung eines Ausführungsbeispiels einer Schaltung zur Auswertung eines Temperatursensors;

Fig. 5　eine schematische Darstellung eines Ausführungsbeispiels einer medizinischen Vorrichtung mit einer bipolaren Elektrodenleitung in einem Testsystem mit Fluidströmung;

Fig. 6　ein beispielhaftes Ablaufdiagramm für ein Verfahren zur Auswertung eines Temperatursignals und Unterbrechung einer Energieabgabe;

Fig. 7　einen beispielhaften Temperatursignalverlauf bei Wandständigkeit und ein Fourier-transformiertes Temperatursignal;

Fig. 8　einen beispielhaften Temperatursignalverlauf bei keiner Wandständigkeit und ein Fourier-transformiertes Temperatursignal;

Fig. 9　ein erstes Beispiel eines Energieabgabeverlaufs, eines Temperatursignalverlaufs und eines Fourier-transformierten Temperatursignals zur Bestimmung der Minimalanforderung an das Temperatursignal für die Auswertung des Temperatursignals;

Fig. 10　ein zweites Beispiel eines Energieabgabeverlaufs, eines Temperatursignalverlaufs und eines Fourier-transformierten Temperatursignals für eine Wandständigkeit einer bipolaren Elektrodenleitung;

Fig. 11　ein drittes Beispiel eines Energieabgabeverlaufs, eines Temperatursignalverlaufs und eines Fourier-transformierten Temperatursignals für keine Wandständigkeit einer bipolaren Elektrodenleitung;

Fig. 12　ein viertes Beispiel eines Energieabgabeverlaufs, eines Temperatursignalverlaufs und eines Fourier-transformierten Temperatursignals für eine Wandständigkeit einer unipolaren Elektrodenleitung;

Fig. 13　ein fünftes Beispiel eines Energieabgabeverlaufs, eines Temperatursignalverlaufs und eines Fourier-transformierten Temperatursignals für keine Wandständigkeit einer unipolaren Elektrodenleitung;

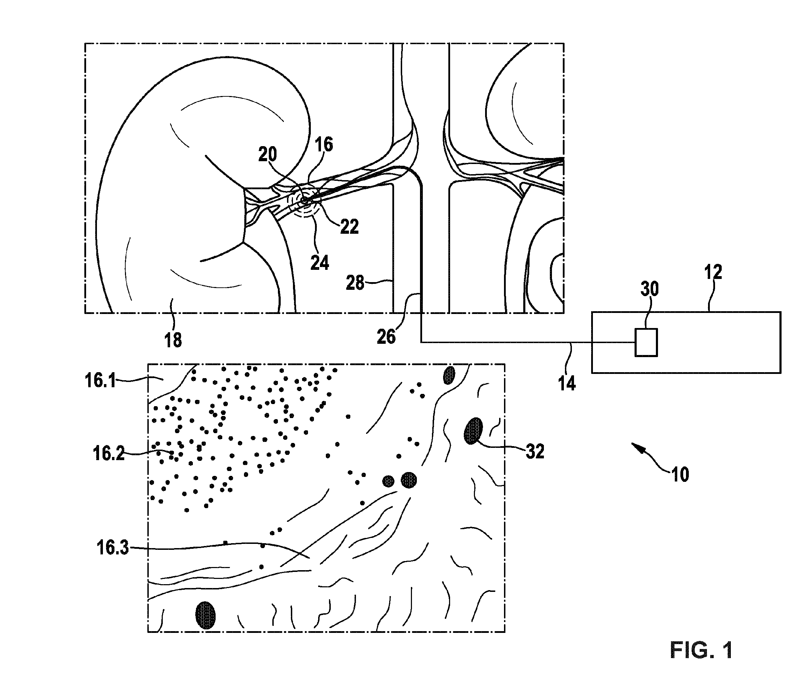

Fig. 1 zeigt ein erstes Ausführungsbeispiel einer medizinischen Vorrichtung 10 mit einer Auswerteeinheit 12 und einer Elektrodenleitung 14, die in eine Renalarterie 16 einer Niere 18 eingeführt ist. Der untere Teil der Figur 1 zeigt einen Teil eines Querschnitts durch ein Gefäß, eine Renalaterie 16 mit den Bereichen inneres Volumen 16.1 des Gefäßes, Media 16.2 und Adventitia 16.3. Ein Temperatursensor 20 liefert ein Temperatursignal der Umgebung einer Ablationselektrode 22, die sich durch eine Energieabgabe 24 der Ablationselektrode 22 verändert. Eine von einem biegeschlaffen Schlauch 26 umschlossene Elektrodenleitung 14 erstreckt sich entlang der Renalarterie 16 und der Aorta 28 bis zur Auswerteeinheit 12 und überträgt das Temperatursignal vom Temperatursensor 20 an die Auswerteeinheit 12. Die Auswerteeinheit 12 wertet periodische Schwankungen eines Signalpegels des Temperatursignals des Temperatursensors 20 aus und erzeugt ein eine Wandständigkeit qualifizierendes Auswertungsausgangsignal in Abhängigkeit davon, ob periodische Schwankungen eines Signalpegels des Temperatursignals unterhalb oder oberhalb eines vorbestimmten Grenzwertes liegen. Die Auswerteeinheit 12 der medizinischen Vorrichtung 10 enthält in diesem Ausführungsbeispiel eine Energiequelle 30. Die Energiequelle 30 versorgt die Elektrodenleitung 14 mit Energie, die zur Energieabgabe 24 an sympathische Nervenfasern 32 und somit zu deren Verödung genutzt werden kann. In diesem Ausführungsbeispiel wird die medizinische Vorrichtung 10 zur Durchführung einer renalen Denervation genutzt.

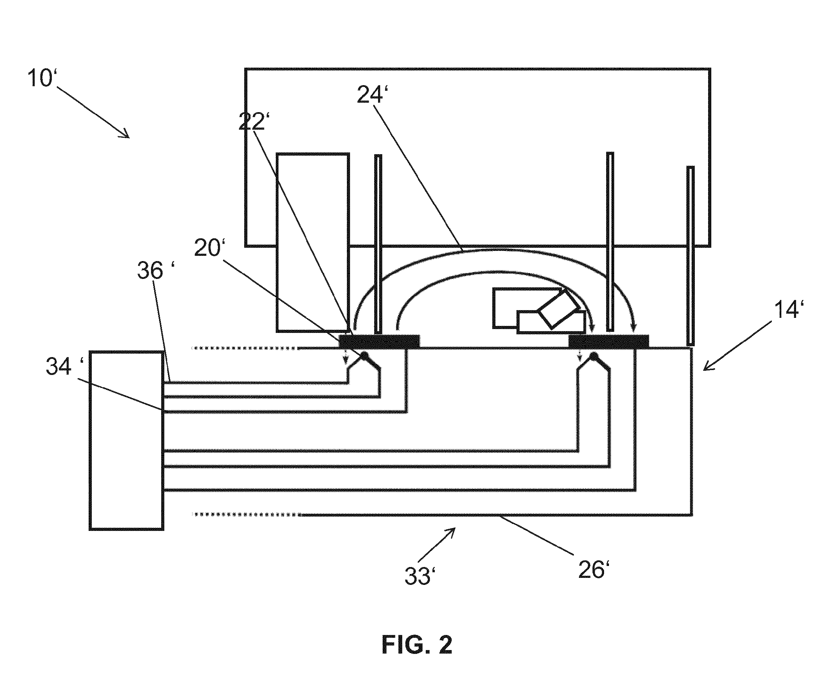

Fig. 2 zeigt als erstes Ausführungsbeispiel der medizinischen Vorrichtung 10' eine bipolare Elektrodenleitung 14'. Die bipolare Elektrodenleitung 14' enthält ein Ablationselektrodenpaar 22' an ihrem distalen Ende 33' und in der Nähe jeder der Ablationselektroden 22' befindet sich ein ihr zugeordneter Temperatursensor 20'. Die Ablationselektroden 22' werden über die elektrischen Leitungen 34' von der Energiequelle 30 mit Energie versorgt und können diese als Energieabgabe 24' abgeben. Die Temperatursensoren 20' sind über Sensorleitungen 36' mit der Auswerteeinheit 12 verbunden und liefern dieser Temperatursignale. Ein biegeschlaffer Schlauch 26' umschließt die elektrischen Leitungen 34' und die Sensorleitungen 36' und isoliert diese elektrisch voneinander. Der biegeschlaffe Schlauch 26' kann auch einen Innenkanal aufweisen, der ein Kühlmittel, zum Beispiel zum Kühlen der Ablationselektroden 22', führen kann (nicht gezeigt). Die Oberfläche der Elektrodenleitung 14' kann eine Beschichtung, beispielsweise mit medizinisch wirksamen Stoffen, haben (nicht gezeigt). Auch kann das distale Ende 33' der Elektrodenleitung 14' ein expandierbarer Ballon oder eine expandierbare Ballonelektrode sein (nicht gezeigt). Auch Elektrodenleitungen in Form von speziellen Kathetern für die renale Denervation sind denkbar (nicht gezeigt). Die Temperatursensoren 20' können beispielsweise als Thermoelemente, Sensorschichten oder dergleichen ausgeführt sein (nicht gezeigt). Die Lage der Temperatursensoren 20' kann auch unabhängig von der Lage der Ablationselektroden 22' sein. Die Lage der Temperatursensoren 20' ist jedoch bevorzugt derart, dass eine durch Energieabgabe von den Ablationselektroden 22' induzierte Erwärmung möglichst vollständig durch die Temperatursensoren 20' erfasst wird. Beispielsweise können Elektrodenleitungen auch einen oder mehrere den Umfang umspannende Temperatursensor(en) aufweisen (nicht gezeigt).

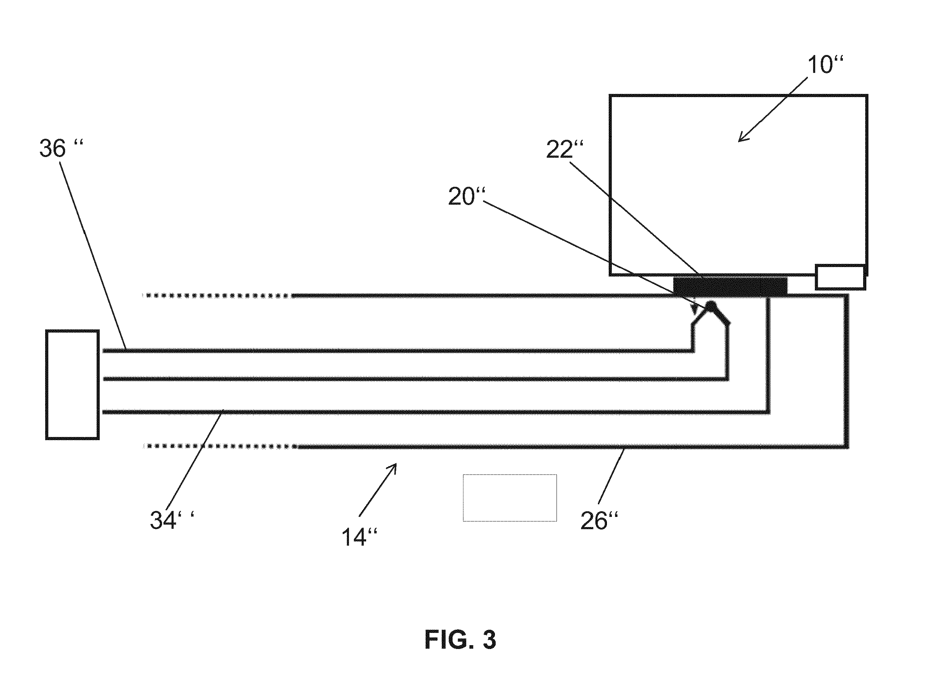

Fig. 3 zeigt als zweites Ausführungsbeispiel der medizinischen Vorrichtung 10" eine unipolare Elektrodenleitung 14". Die eine Ablationselektrode 22" kann mit Hilfe einer entfernt angeordneten Gegenelektrode betrieben werden, um den Stromkreis zu schließen (nicht gezeigt). Die Ablationselektrode 22" wird in diesem Fall über die elektrische Leitung 34" mit Energie von der Energiequelle 30 versorgt. Der Temperatursensor 20" ist in der Nähe der Ablationselektrode 22" angeordnet und über die Sensorleitungen 36" mit der Auswerteeinheit 12 verbunden, an die der Temperatursensor 20" ein Temperatursignal liefert. Ein biegeschlaffer Schlauch 26" umschließt die elektrische Leitung 34" und die Sensorleitungen 20" und isoliert die Leitungen elektrisch voneinander.

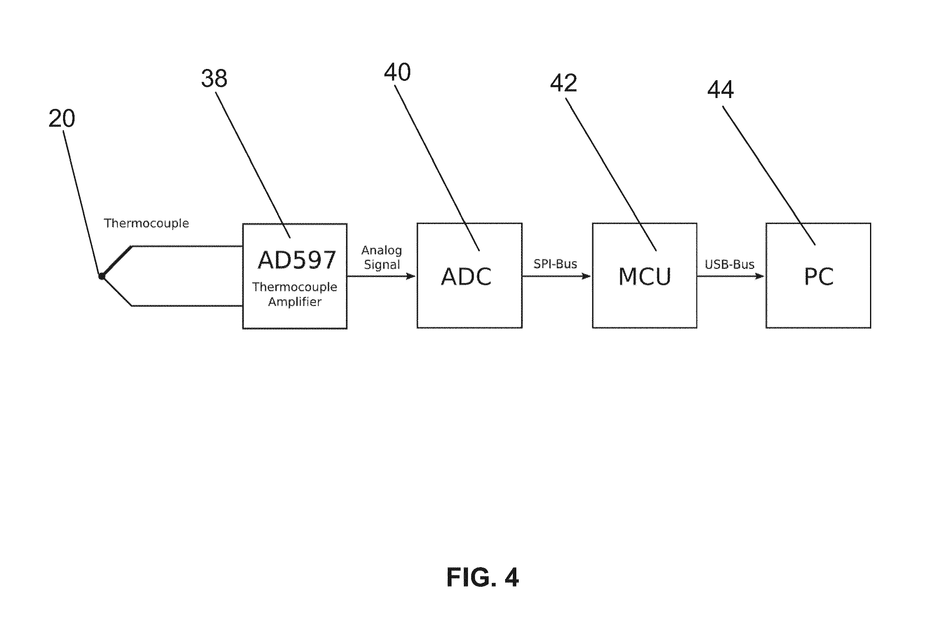

Fig. 4 zeigt einen beispielhaften Schaltkreis zur Auswertung eines Temperatursignals eines Temperatursensors 20. Der Temperatursensor 20 liefert ein Temperatursignal an einen Temperatursignalverstärker 38, der das Temperatursignal mit integrierter Vergleichsstellenkompensation verstärkt, in diesem Ausführungsbeispiel ist der Temperatursignalverstärker 38 ein AD597. Der Temperatursignalverstärker 38 liefert das analoge Temperatursignal an einen Analog-zu-Digital-Wandler 40. Der Analog-zu-Digital-Wandler 40 wandelt das analoge Temperatursignal in ein digitales Temperatursignal und liefert es an eine Mikrocontrollereinheit 42. Die Mikrocontrollereinheit 42 kodiert das digitale Temperatursignal in eine computerlesbare Form und liefert es an eine Computereinheit 44. In der Computereinheit 44 kann das digitale Temperatursignal ausgewertet werden. In einem alternativen Ausführungsbeispiel kann anstatt der Mikrocontrollereinheit 42 und der Computereinheit 44 auch ein Ablationsgenerator im Schaltkreis integriert sein (nicht gezeigt).

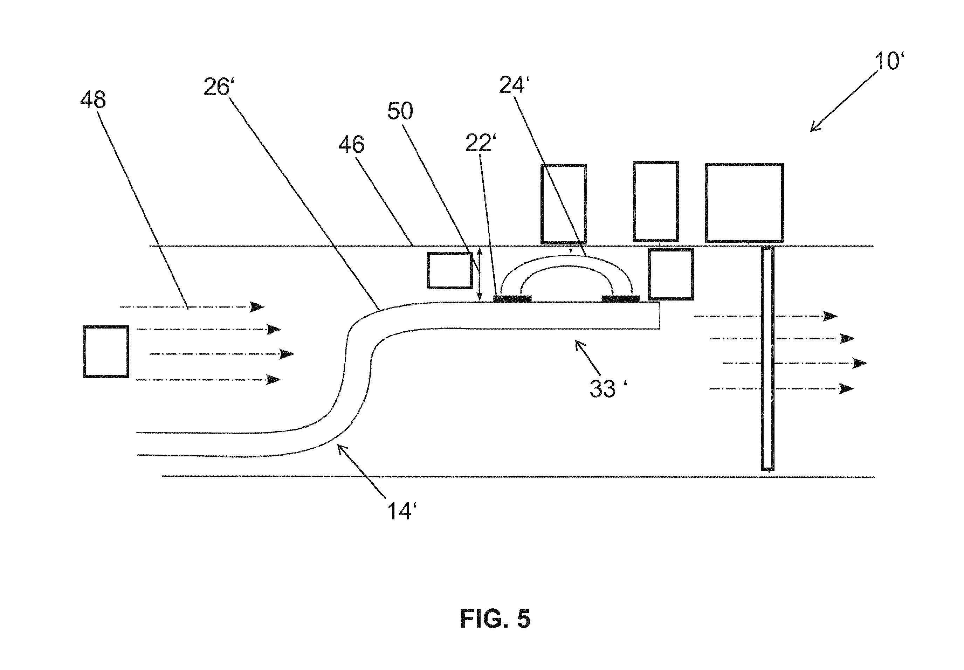

Fig. 5 zeigt als weiteres Ausführungsbeispiel eine medizinische Vorrichtung 10" in Form einer bipolaren Elektrodenleitung 14' die in einem röhrenförmigen Hohlraum mit Wand 46 angeordnet ist. Der röhrenförmige Hohlraum weist eine Fluidströmung 48 auf, die koaxial zur Achse der Elektrodenleitung 14' gerichtet ist. Das distale Ende 33' der Elektrodenleitung 14' befindet sich in der Nähe der Wand 46. Die Ablationselektroden 22' haben einen Wandabstand 50, der die Energieabgabe 24' an die Wand 50 verringert. Ein Teil der durch die Energieabgabe 24' erzeugten Wärme wird von der Fluidströmung 48 weggespült. Die Fluidströmung 48 kann periodisch gepulst sein, wodurch ein von den Temperatursensoren 20' gemessener Temperaturverlauf die Periodizität der Fluidströmung wiederspiegelt.

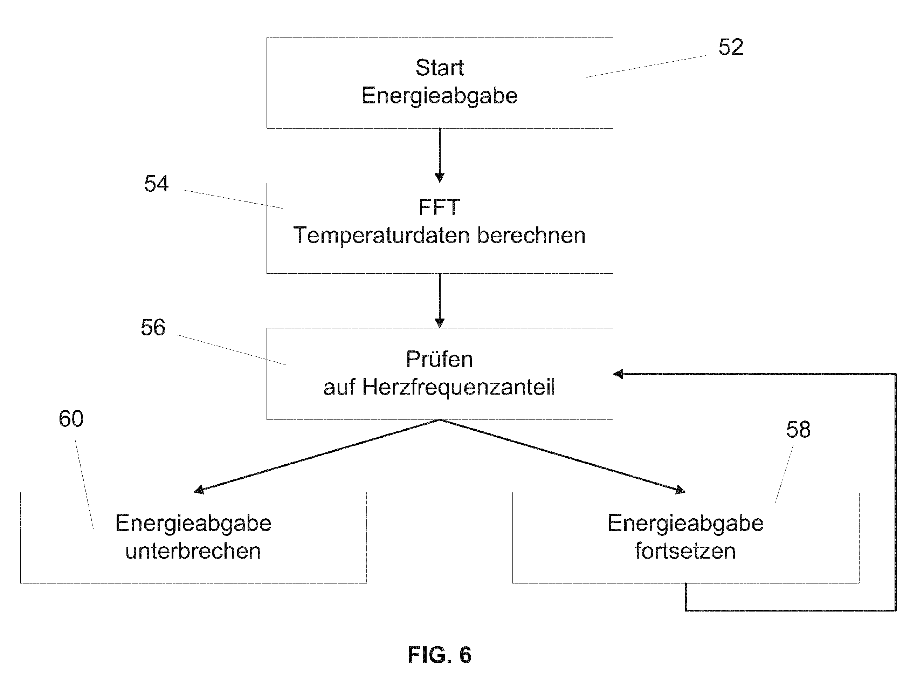

Fig. 6 zeigt ein beispielhaftes Ablaufdiagramm für ein Verfahren, dass die folgenden Schritte umfasst:

Schritt 52:

Starten einer Energieabgabe 24, beispielsweise von der Energiequelle 30 über eine unipolare 14" oder bipolare Elektrodenleitung 14' an eine Wand 46.

Schritt 54:

Das beispielsweise von dem Temperatursensor 20 gelieferte Temperatursignal wird in diesem Ausführungsbeispiel mittels einer Fast-Fourier-Transformation z. B. in der Auswerteeinheit 12 in den Frequenzbereich transformiert. Auch andere Zeit-Frequenz-Transformationen, wie beispielsweise eine Wavelet-Transformation, Short-Time-Fourier-Transformation oder dergleichen sind denkbar, die das Temperatursignal aus der Zeitdomäne in den Frequenzbereich transformieren. Bevorzugt werden im Mittel wenigstens 3,3 Temperaturmesswerte pro Sekunde, besonders bevorzugt wenigstens 6,6 Temperaturmesswerte pro Sekunde und insbesondere bevorzugt wenigstens 22 Temperaturmesswerte pro Sekunde genutzt um die Zeit-Frequenz-Transformation des Temperatursignals durchzuführen. Die Aufgabe des Schrittes 54 ist es das Temperatursignal derart vorzubereiten, dass in dem folgenden Schritt 56 eine Auswertung des Temperatursignals durch Vergleich mit vorbestimmten Grenzwerten möglich wird, daher kann auch jede andere Möglichkeit zur Vorbereitung des Temperatursignals denkbar sein.

Schritt 56:

In diesem Ausführungsbeispiel wird das Fourier-transformierte Temperatursignal beispielsweise in der Auswerteeinheit 12 ausgewertet, indem die Temperaturamplituden eines Frequenzbereichs von beispielsweise 0,2 bis 4,2 Hz, bevorzugt 0,8 bis 3,4 Hz, insbesondere bevorzugt 1,1 bis 2,0 Hz mit einem vorbestimmten Grenzwert der Temperaturamplitude verglichen werden. Der Grenzwert in diesem Ausführungsbeispiel entspricht bevorzugt 0,6° C für die bipolare Elektrodenleitung 14', wobei ein Grenzwert unter 0,6° C eine Wandständigkeit der bipolaren Elektrodenleitung 14' anzeigt. Für die unipolare Elektrodenleitung 14" entspricht der Grenzwert in diesem Ausführungsbeispiel bevorzugt 0,2° C, wobei ein Grenzwert oberhalb von 0,2° C eine Wandständigkeit der unipolaren Elektrodenleitung 14" anzeigt. Ist eine Wandständigkeit vorhanden, wird Schritt 58 ausgeführt, falls keine Wandständigkeit vorhanden ist, wird anstatt dessen Schritt 60 ausgeführt.

Schritt 58:

Die Energieabgabe 24 wird da eine Wandständigkeit, beispielsweise der Elektrodenleitung 14 ermittelt wurde fortgesetzt und das Verfahren springt zu Schritt 54 um weitere Temperatursignale zu analysieren. Die Energieabgabe 24 kann in diesem Fall so lange fortgesetzt werden, bis die Energiequelle 30 manuell abgeschaltet wird oder keine Wandständigkeit im Schritt 56 festgestellt wird, was zu Schritt 60 führt.

Schritt 60:

Die Energieabgabe 24 wird unterbrochen sobald keine Wandständigkeit beispielsweise der Elektrodenleitung 14 festgestellt wird. Hierfür kann beispielsweise in der Auswerteeinheit 12 ein Energiequellenabschaltsignal erzeugt werden, welches im Betrieb ein Abschalten der Energieabgabe 24 für eine Ablation bewirkt. Das Energiequellenabschaltsignal kann zum Abschalten bzw. Unterbrechen der Energiequelle 30 von der Auswerteeinheit 12 an die Energiequelle 30 oder einer Steuereinheit der Energiequelle 30 übermittelt werden, woraufhin diese die Energieabgabe 24 an die Elektrodenleitung 14 abschaltet bzw. unterbricht. Die medizinische Vorrichtung 10, auf der das in Fig. 6 gezeigte Verfahren verwendet werden kann, muss in diesem Fall manuell neu gestartet werden. Vorher ist sicherzustellen, dass beispielsweise die Elektrodenleitung 14 eine Wandständigkeit aufweist, indem ihre Lage verändert wird, da ansonsten ein Starten der Energieabgabe 24 nach Schritt 52 zu einer erneuten Unterbrechung der Energieabgabe 24 durch die in Schritt 56 festgestellte fehlende Wandständigkeit führt.

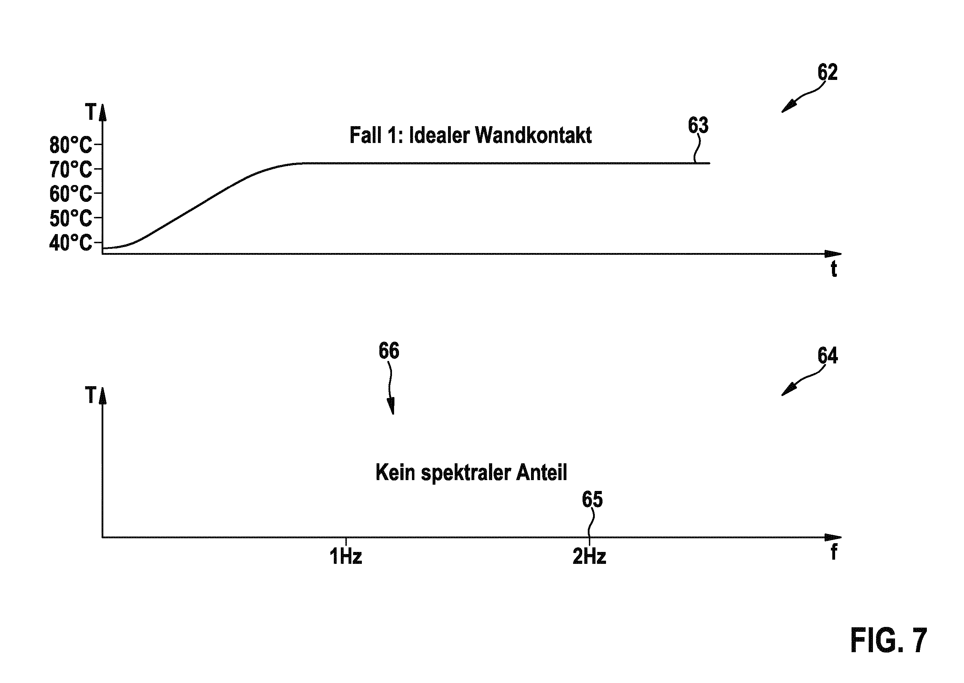

Fig. 7 zeigt einen Temperatursignalverlauf 62 mit dem Temperatursignals 63 und einen in den Frequenzbereich transformierten Temperatursignalverlauf 64 mit einem Zeit-Frequenz-transformierten Temperatursignal 65 für den Fall eines idealen Wandkontaktes einer bipolaren Elektrodenleitung 14'. Ein bevorzugt zu analysierender Frequenzbereich 66 des Zeit-Frequenz-transformierten Temperatursignals 65 um 1 Hz zeigt keine Amplitude, somit liegt der Wert unterhalb des vorbestimmten bevorzugten Grenzwertes von 0,6° C und die Auswertung liefert daher ein eine Wandständigkeit anzeigendes Auswertungsausgangsignal. Der bevorzugte zu analysierende Frequenzbereich 66 liegt beispielsweise zwischen 0 Hz (0 bpm (beats per minute)) und 20 Hz (1200 bpm), bevorzugt zwischen 0,2 Hz (12 bpm) und 4,2 Hz (252 bpm), besonders bevorzugt zwischen 0,8 Hz (48 bpm) und 3,4 Hz (204 bpm) und insbesondere bevorzugt zwischen 1,1 Hz (66 bpm) und 2,0 Hz (120 bpm).

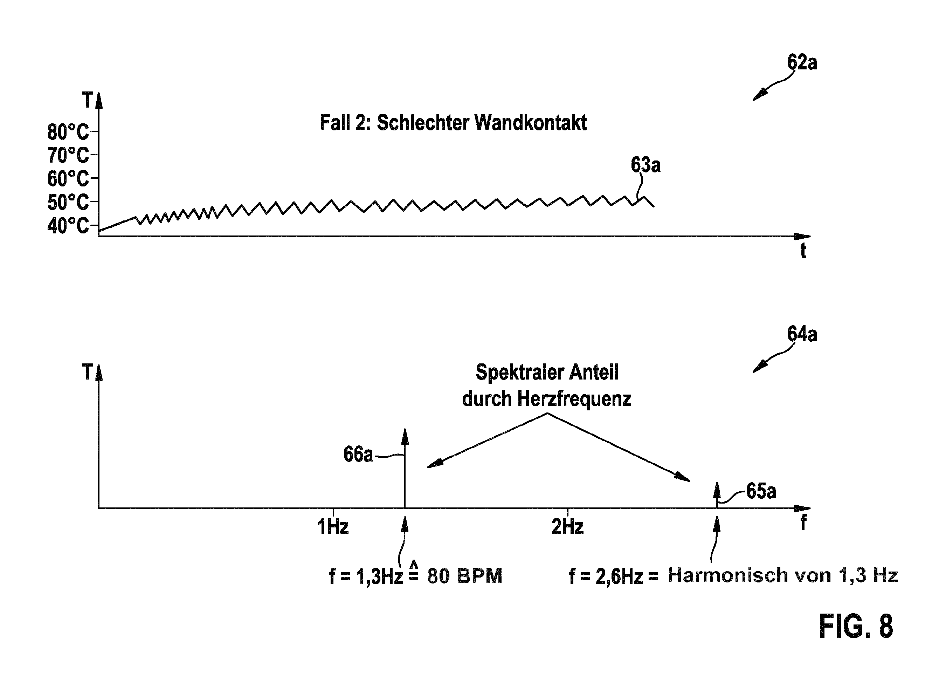

Fig. 8 zeigt ein Temperatursignalverlauf 62a mit dem Temperatursignal 63a und einen in den Frequenzbereich transformierten Temperatursignalverlauf 64a mit dem Zeit-Frequenz-transformierten Temperatursignal 65a für den Fall eines schlechten Wandkontaktes einer bipolaren Elektrodenleitung 14'. Ein bevorzugt zu analysierender Frequenzbereich 66a um 1 Hz, sowie ein weiterer Frequenzbereich um 2 Hz zeigen eine Amplitude mit einem Wert oberhalb des vorbestimmten bevorzugten Grenzwertes von 0,6° C. Die Auswertung liefert daher ein keine Wandständigkeit der bipolaren Elektrodenleitung 14' anzeigendes Auswertungsausgangsignal.

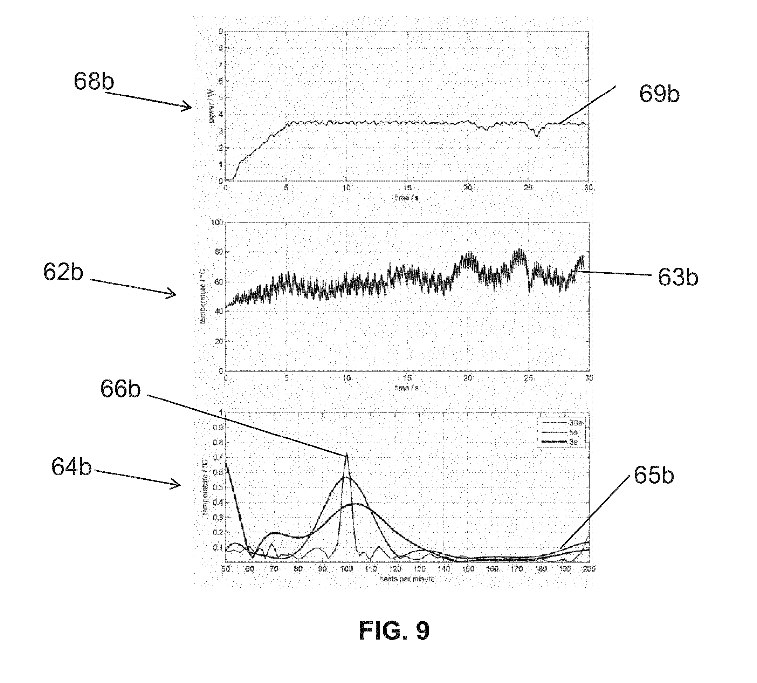

Fig. 9 zeigt den Leistungsverlauf 68b mit dem Leistungssignal 69b, den Temperatursignalverlauf 62b mit dem Temperatursignal 63b und einen in den Frequenzbereich transformierten Temperatursignalverlauf 64b mit dem Zeit-Frequenz-transformierten Temperatursignal 65b. Der Leistungsverlauf 68b zeigt einen kontinuierlichen Anstieg des Leistungssignals 69b zwischen t=0 s bis t=5 s, der sich im Temperatursignalverlauf 62b in einer Erhöhung des Temperatursignals 63b auswirkt. Im weiteren zeitlichen Verlauf wird das Leistungssignal 69b konstant gehalten. Das Temperatursignal 63b zeigt periodische Schwankungen mit verschiedenen Frequenzen. Der in den Frequenzbereich transformierte Temperatursignalverlauf 64b ist über bpm (beats per minute), also als Spektrum, aufgetragen, wobei sich das Spektrum des Temperatursignalverlaufs von 0,833 Hz (50 bpm) bis 3,333 Hz (200 bm) erstreckt. Drei verschiedene Aufnahmedauern des Temperatursignals 63b, die in den Frequenzbereich transformiert wurden um ein Zeit-Frequenz-transformiertes Temperatursignal 65b zu erzeugen sind dargestellt, um zu ermitteln, welche Aufnahmedauer mindestens notwendig ist, um ein Zeit-Frequenz-transformiertes Temperatursignal 65b zu erhalten, mit dem eine Wandständigkeit einer Elektrodenleitung 14 bestimmt werden kann. Ein Peak des Zeit-Frequenz-transformierten Temperatursignal 65b ist im bevorzugten Frequenzbereich 66b um 1,667 Hz (100 bpm) zu erkennen. Der Peak kann verwendet werden, um eine Wandständigkeit zu bestimmen. Auch eine Güte der Wandständigkeit bzw. eine grobe Entfernung zwischen Wand 46 und Elektrodenleitung 14 kann über den maximalen Temperaturwert des Zeit-Frequenz-transformierten Temperatursignal 65b, beispielsweise im bevorzugten Frequenzbereich 66b abgeschätzt werden.

Um das analoge Temperatursignal 63b zu digitalisieren, muss eine Anzahl von Temperaturwerten pro Zeiteinheit erfasst werden. Eine Abtastfrequenz $f_s$ (Samplingrate) zum Digitalisieren der Temperaturdaten soll dabei mindestens doppelt so groß wie die maximal im Temperatursignal 63b auftretende Frequenz sein. Beispielsweise kann die maximale Frequenz durch eine Pulsfrequenz eines Menschen vorgegeben sein, beispielsweise mit Werten bis zu 3,333 Hz (200 bpm). Die Abtastfrequenz soll in diesem Fall also mindestens 6,667 Hz (400 bpm) betragen. Bevorzugt wird die Abtastfrequenz $f_s$ größer gewählt, um Aliasing-Effekte zu vermeiden, beispielsweise mit bis zu 22 Hz, bevorzugt mit bis zu 44 Hz, besonders bevorzugt mit bis zu 100 Hz. Die Transformation der digitalisierten Temperaturdaten in den Frequenzbereich erfolgt dann beispielsweise mit Hilfe eines Algorithmus' für Zeit-Frequenz-Transformation, beispielsweise Fast-Fourier-Transformation (FFT) oder dergleichen. Je kleiner die Frequenzauflösung dabei sein soll, desto größer muss die Anzahl N an Messwerten gewählt werden, die in die Berechnung einfließen. Die Anzahl N der Temperaturmesswerte folgt aus der Auflösung $\Delta f$ im Frequenzbereich mit $N = \dfrac{f_s}{\Delta f}$. Hieraus folgt die Zeit T für das Erfassen von N Datenwerten mit $T = N \cdot \dfrac{1}{f_s}$. Bei einer Abtastfrequenz von 22 Hz ist also für eine Pulsfrequenzauflösung von 10 bpm eine Aufzeichnung über 6 s notwendig. Eine möglichst schnelle Detektion der Pulsfrequenz in den Temperaturdaten ist vorteilhaft. Es hat sich in praktischen Versuchen gezeigt, dass innerhalb von 3 s nach Beginn einer Energieabgabe bestimmt werden kann, ob ein Anteil der Pulsfrequenz in den Daten vorhanden ist.

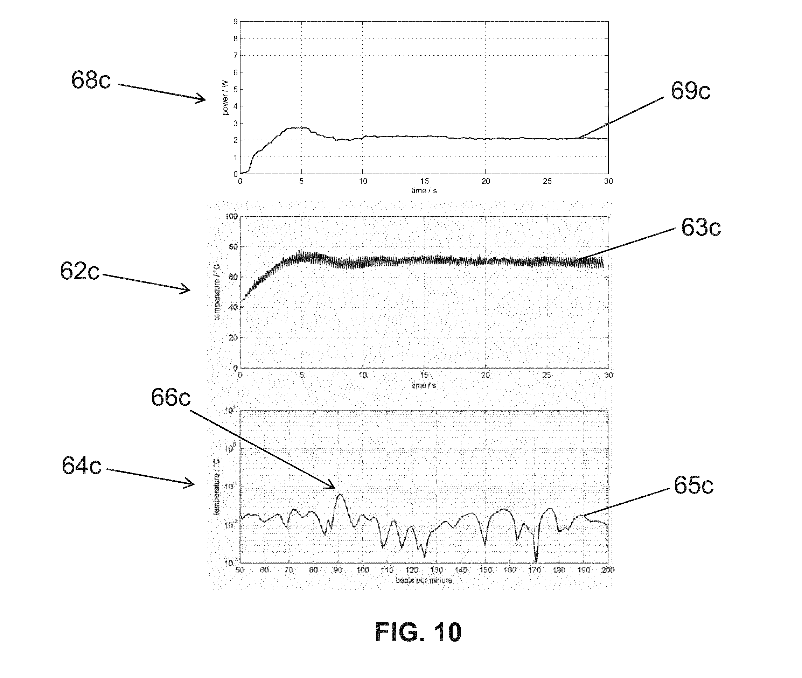

Fig. 10 zeigt beispielhafte Messungen für eine bipolare Elektrodenleitung 14' mit einer Wandständigkeit. Ein Temperatursignal 63c wird in diesem Ausführungsbeispiel nur für den Temperatursensor 20' abgebildet, der das höhere Temperatursignal 63c zeigt. Es ist auch denkbar, dass ein Mittelwert aus den Temperatursignalen der Temperatursensoren 20' gebildet wird oder alle Temperatursignale individuell ausgewertet werden. Im Gegensatz zu Fig. 9 sind die Werte der Temperatur-Achse des in den Frequenzbereich transformierten Temperatursignalverlaufs 64c logarithmisch aufgetragen. Die übrigen Messungen oder Spektren folgen der Auftragung der Messungen oder Spektren der Fig. 9. Der Leistungsverlauf 68c zeigt einen kontinuierlichen Anstieg des Leistungssignals 69c bis ca. t=5 s. Danach pendelt sich das Leistungssignal auf ca. 2 W ein. Das Temperatursignal 63c zeigt einen relativ starken mit dem im Leistungssignal 69c dargestellten Anstieg der Energieabgabe 24' korrelierten Anstieg der Temperatur bis ca. t=5 s. Im weiteren Zeitverlauf zeigt das Temperatursignal 63c eine periodische Oszillation um ca. 70° C. Die Analyse der periodischen Schwingung durch Transformation von der Zeitdomäne in den Frequenzbereich zeigt im Temperaturspektrum 64c einen relativ flachen Verlauf des Zeit-Frequenz-transformierten Temperatursignals 65c. Das Maximum des Zeit-Frequenz-transformierten Temperatursignals 65c wird im bevorzugten Frequenzbereich 66c bei ca. 1,533 Hz (92 bpm) erreicht. Das Maximum in diesem beispielhaften Temperaturspektrum 64c hat einen Wert von ca. 0.065° C und liegt damit unter einem vorbestimmten Grenzwert zur Erfassung, ob eine Wandständigkeit vorliegt. Der vorbestimmte Grenzwert entspricht bevorzugt einem Wert zwischen 0,1° C und 1,0° C, besonders bevorzugt einem Wert zwischen 0,2° C und 0,8° C und insbesondere bevorzugt einem Wert zwischen 0,3° C und 0,6° C. Somit zeigt das Temperaturspektrum 64c ein eine Wandständigkeit der Elektrodenleitung 14' anzeigendes Auswertungsausgangsignal.

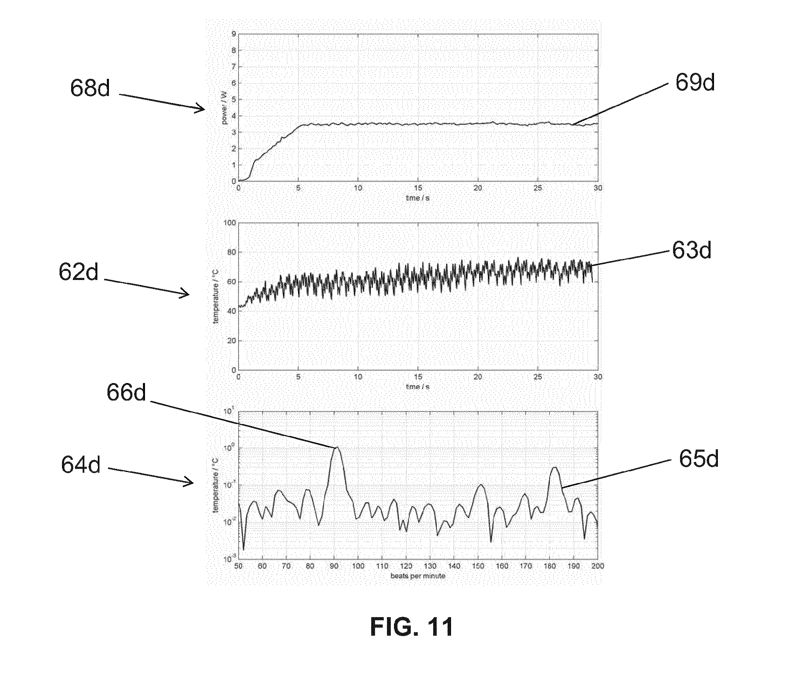

Weitere beispielhafte Messungen haben gezeigt, dass das Erreichen einer Zieltemperatur von 70° C zu einem Maximalwert von 0,08° C im über die Frequenz aufgetragenen Temperaturspektrum führt, wenn eine Wandständigkeit der Elektrodenleitung 14' vorliegt. Fig. 11 zeigt beispielhafte Messungen für eine bipolare Elektrodenleitung 14' mit keiner Wandständigkeit. Im Gegensatz zu Fig. 10 zeigt der Verlauf des Temperatursignals 63d im Temperatursignalverlauf 62d eine größere Schwankung. Der Leistungsverlauf 68d zeigt ein etwas höher liegendes Leistungssignal 69d im Vergleich zu dem Leistungssignal 69c in Fig. 10, d. h. das Ablationselektrodenpaar 22' hat eine höhere Energieabgabe 24'. Das über die Frequenz aufgetragene Temperaturspektrum 64d zeigt einen stärker schwankenden Verlauf des Zeit-Frequenz-transformierten Temperatursignals 65d im Vergleich zu dem Zeit-Frequenz-transformierten Temperatursignal 65c. Insbesondere im bevorzugt zu analysierenden Frequenzbereich 66d bildet sich ein Peak heraus, der mit einem Wert von ca. 1,0° C über dem vorbestimmten Grenzwert liegt. Somit zeigt das Temperaturspektrum 64d ein keine Wandständigkeit der Elektrodenleitung 14' anzeigendes Auswertungsausgangsignal.

Weitere beispielhafte Messungen haben gezeigt, dass das Nicht-Erreichen einer Zieltemperatur von 70° C zu einem Maximalwert von 0,6° C bis 1,0° C im über die Frequenz aufgetragenen Temperaturspektrum führt, wenn keine Wandständigkeit der Elektrodenleitung 14' vorliegt.

Zusammenfassen der Ergebnisse aus Fig. 10 und Fig. 11 führt zu der Erkenntnis, dass ein guter Wandkontakt bzw. eine Wandständigkeit bei einem Grenzwert unterhalb von 0,6° C vorliegen kann. Allgemein kann man eine Wandständigkeit einer bipolaren Elektrodenleitung 14' daran erkennen, dass kein erhöhter Temperaturwert eines Frequenzbereichs erkennbar ist, der der periodischen Fluidströmung entspricht. Der Fluidstrom, beispielsweise Blutstrom in einer Renalarterie kann im Temperatursignal in Abhängigkeit der Entfernung zwischen Wand und Elektrodenleitung bzw. Ablationselektrodenpaar erkannt werden, da die Wärme die bei der Energieabgabe entsteht von dem Fluidstrom weggespült wird und somit periodisch die Höhe des Temperatursignals senkt. Bei größerer Entfernung zur Wand, d. h. keine Wandständigkeit ist die Temperaturschwankung höher, da mehr Wärme an das Fluid abgegeben wird. Der Fluidstrom kann auch eine Varianz in der Periodizität aufweisen, beispielsweise bei einem sich kontinuierlich ändernden Herzrhythmus. Die Varianz kann dabei zu einer Verbreiterung des bevorzugt zu analysierenden Frequenzbereichs führen.

Die Erfassung der Wandständigkeit kann beispielsweise genutzt werden um einen Ablationsgenerator oder eine Energiequelle abzuschalten, wenn daran angeschlossene Ablationselektroden nicht im Kontakt mit einer Wand sind (nicht gezeigt).

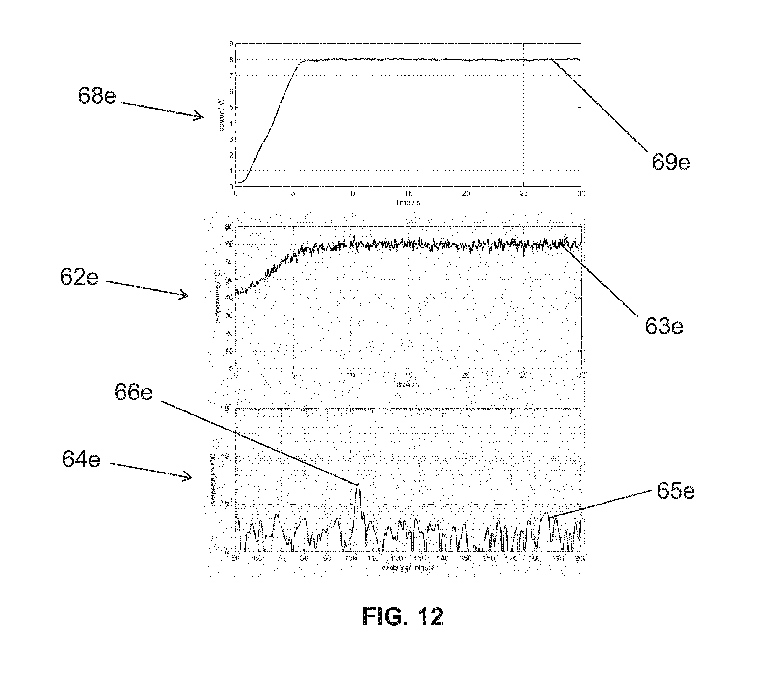

Fig. 12 zeigt beispielhafte Messungen für eine unipolare Elektrodenleitung 14" mit einer Wandständigkeit. Ein Temperatursignal 63e wird in diesem Ausführungsbeispiel im Temperatursignalverlauf 62e für den Temperatursensor 20" abgebildet. Der Leistungsverlauf 68e zeigt einen kontinuierlichen Anstieg des Leistungssignals 69e bis ca. t=6 s. Im weiteren zeitlichen Verlauf wird die Energieabgabe 24" konstant gehalten. Das Temperatursignal 63e folgt dem Anstieg des Leistungssignals 69e bis t=6 s und oszilliert danach um einen Wert von ca. 70° C. Die Zeit-Frequenz-Transformation des Temperatursignals 63e in den Frequenzbereich liefert ein Temperaturspektrum 64e, dessen Zeit-Frequenztransformiertes Temperatursignal 65e in einem bevorzugten Frequenzbereich 66e einen Peak von ca. 0,26° C zeigt.

Bei der Verwendung einer unipolaren Elektrodenleitung 14" wird zusätzlich eine Neutralelektrode bzw. Klebeelektrode außerhalb des Fluidströmungssystems, beispielsweise am Körper, befestigt. Die Fläche einer solchen Neutralelektrode ist typischerweise um ein Vielfaches größer, als die Fläche der Ablationselektrode 22". Im Gegensatz zur bipolaren Elektrodenleitung 14' ist eine periodische Schwingung gut sichtbar im Zeit-Frequenz-transformierten Temperatursignal 65e erkennbar. In diesem Fall zeigt dies jedoch eine Wandständigkeit der unipolaren Elektrodenleitung 14" an, was durch die Höhe des Temperatursignals unterstützt wird. Somit zeigt das Temperaturspektrum 64e ein eine Wandständigkeit der unipolaren Elektrodenleitung 14" anzeigendes Auswertungsausgangsignal.

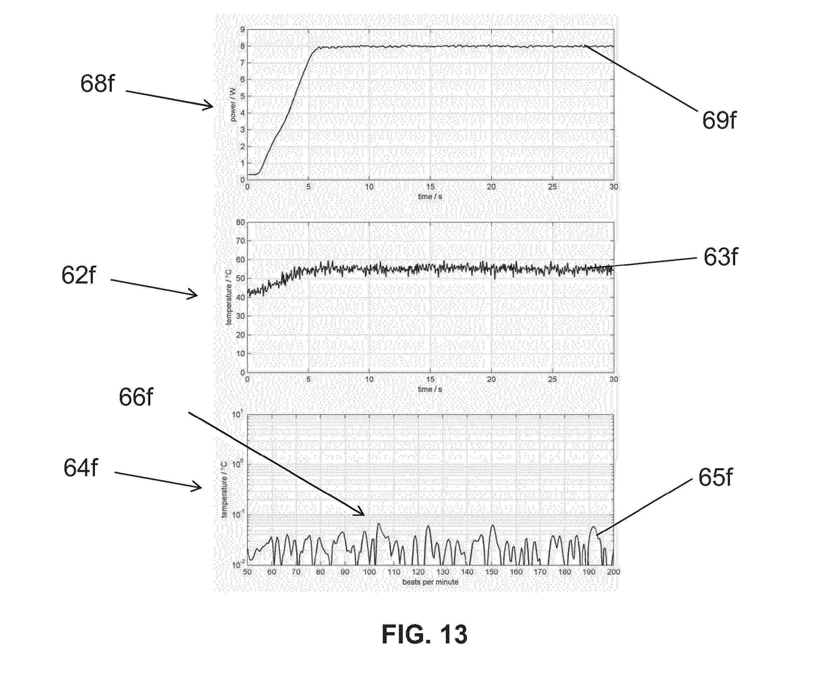

Fig. 13 zeigt beispielhafte Messungen für eine unipolare Elektrodenleitung 14" mit keiner Wandständigkeit. Der Verlauf des Leistungssignals 69f im Leistungsverlauf 68f ist weitgehend identisch mit dem Leistungssignal 69e aus Fig. 12. Der Verlauf des Temperatursignals 63f im Temperatursignalverlauf 62f ist jedoch flacher und erreicht nur eine maximale Höhe von ca. 60° C. Das Temperatursignal 63f oszilliert um einen Wert von ca. 55° C. Die Zeit-Frequenz-Transformation liefert im Temperaturspektrum 64f keine starken Schwankungen des Zeit-Frequenz-transformierten Temperatursignals 65f, das auch im bevorzugten Frequenzbereich 66f keinen nennenswerten Peak hat. In Verbindung mit der geringeren Temperatur von ca. 55° C kann darauf geschlossen werden, dass in diesem Fall ein schlechterer Wandkontakt besteht, bzw. dass die unipolare Elektrodenleitung 14" einen größeren Wandabstand 50 zur Wand 46 hat, als in Fig. 12. Somit zeigt das Temperaturspektrum 64f ein keine Wandständigkeit der unipolaren Elektrodenleitung 14" anzeigendes Auswertungsausgangsignal.

Weitere beispielhafte Messungen haben gezeigt, dass das Nicht-Erreichen einer Zieltemperatur von 70° C zu einem Maximalwert von 0,1° C im Temperaturspektrum in Frequenzabhängigkeit führt, wenn keine Wandständigkeit der Elektrodenleitung 14" vorliegt, während eine Wandständigkeit für Maximalwerte oberhalb von 0,2° C gefunden werden kann.

Zur Messung eines Wandkontakts vor einer längeren Energieabgabe 24, kann eine geringere Energiemenge abgegeben werden. Bevorzugt wird dies nur für wenige Sekunden, beispielsweise 6 s, bevorzugt 3 s durchgeführt, um eine Wandständigkeit festzustellen oder gegebenenfalls die Lage der Elektrodenleitung 14 zu justieren.

Bezugszeichenliste

| | |
|---|---|
| 10 | medizinische Vorrichtung |
| 12 | Auswerteeinheit |
| 14 | Elektrodenleitung |
| 16 | Renalarterie |
| 18 | Niere |
| 20 | Temperatursensor |
| 22 | Ablationselektrode |
| 24 | Energieabgabe |
| 26 | biegeschlaffer Schlauch |
| 28 | Aorta |
| 30 | Energiequelle |
| 32 | sympathische Nervenfasern |
| 33 | distales Ende der Elektrodenleitung |
| 34 | elektrische Leitung |
| 36 | Sensorleitung |
| 38 | Temperatursignalverstärker |
| 40 | Analog-zu-Digital-Wandler |
| 42 | Mikrocontrollereinheit |
| 44 | Computereinheit |
| 46 | Wand |
| 48 | Fluidströmung |
| 50 | Wandabstand |
| 62 | Temperatursignalverlauf in Zeitabhängigkeit |
| 63 | Temperatursignal |
| 64 | In den Frequenzbereich transform. Temperatursignalverlauf (Temperaturspektrum) |
| 65 | Zeit-Frequenz-transformiertes Temperatursignal |
| 66 | bevorzugter Frequenzbereich für Wandständigkeitserfassung |
| 68 | Leistungsverlauf in Zeitabhängigkeit |
| 69 | Leistungssignal |

**Patentansprüche**

1.  Medizinische Vorrichtung (10; 10'; 10"; 10''') mit einer Auswerteeinheit (12) und mit einer Elektrodenleitung (14; 14'; 14"), die wenigstens einen Temperatursensor (20; 20'; 20") umfasst, der ausgebildet ist ein Temperatursignal (63; 63a; 63b; 63c; 63d; 63e; 63f) an die Auswerteeinheit (12) zu liefern, wobei die Auswerteeinheit (12) ausgebildet ist, periodische Schwankungen eines Signalpegels des Temperatursignals (63; 63a; 63b; 63c; 63d; 63e; 63f) auszuwerten und ein eine Wandständigkeit der Elektrodenleitung (14; 14'; 14") qualifizierendes Auswertungsausgangsignal in Abhängigkeit davon zu erzeugen, ob periodische Schwankungen eines Signalpegels des Temperatursignals (63; 63a; 63b; 63c; 63d; 63e; 63f) unterhalb oder oberhalb eines vorbestimmten Grenzwertes liegen.

2.  Medizinische Vorrichtung (10; 10'; 10"; 10''') nach Anspruch 1, wobei die Elektrodenleitung (14; 14'; 14") eine bipolare Elektrodenleitung (14') mit einem Ablationselektrodenpaar (22') für eine bipolare Ablation ist und die Auswerteeinheit (12) ein eine Wandständigkeit der Elektrodenleitung (14') anzeigendes Auswertungsausgangsignal erzeugt, wenn periodische Schwankungen eines Signalpegels des Temperatursignals (63c) unterhalb des vorbestimmten Grenzwertes liegen und/oder ein keine Wandständigkeit der Elektrodenleitung (14') anzeigendes Auswertungsausgangsignal erzeugt, wenn periodische Schwankungen eines Signalpegels des Temperatursignals (63d) oberhalb des vorbestimmten Grenzwertes liegen.

3.  Medizinische Vorrichtung (10; 10'; 10"; 10''') nach Anspruch 2, wobei der vorbestimmte Grenzwert einem Wert zwischen 0,1° C und 1,0° C, bevorzugt einem Wert zwischen 0,2° C und 0,8° C, insbesondere bevorzugt einem Wert zwischen 0,3° C und 0,6° C entspricht.

4.  Medizinische Vorrichtung (10; 10'; 10"; 10''') nach Anspruch 1, wobei die Elektrodenleitung (14; 14'; 14") eine unipolare Elektrodenleitung (14") mit einer Ablationselektrode (22") zur unipolaren Ablation ist und die Auswerteeinheit (12) ein eine Wandständigkeit der Elektrodenleitung (14") anzeigendes Auswertungsausgangsignal erzeugt, wenn periodische Schwankungen eines Signalpegels des Temperatursignals (63e) oberhalb des vorbestimmten Grenzwertes liegen und/oder ein keine Wandständigkeit der Elektrodenleitung (14") anzeigendes Auswertungsausgangsignal erzeugt, wenn periodische Schwankungen eines Signalpegels des Temperatursignals (63f) unterhalb des vorbestimmten Grenzwertes liegen.

5.  Medizinische Vorrichtung (10; 10'; 10"; 10''') nach Anspruch 4, wobei der vorbestimmte Grenzwert einem Wert zwischen 0,05° C und 0,6° C, bevorzugt einem Wert zwischen 0,1° C und 0,4° C, insbesondere bevorzugt einem Wert zwischen 0,2° C und 0,3° C entspricht.

6.  Medizinische Vorrichtung (10; 10'; 10"; 10''') nach wenigstens einem der Ansprüche 2 bis 5, wobei der wenigstens eine Temperatursensor (20; 20'; 20") in der Nähe der wenigstens einen Ablationselektrode (22; 22'; 22") angeordnet ist.

7.  Medizinische Vorrichtung (10; 10'; 10"; 10''') nach wenigstens einem der Ansprüche 2 bis 6, wobei die medizinische Vorrichtung (10; 10'; 10"; 10''') eine Energiequelle (30) aufweist, die mit der Elektrodenleitung (14; 14'; 14") und der Auswerteeinheit (12) elektrisch verbunden ist und ausgebildet ist Energie an die Elektrodenleitung (14; 14'; 14") abzugeben und wobei die Auswerteeinheit (12) ausgebildet ist, ein die Energiequelle (30) abschaltendes Energiequellenabschaltsignal zu erzeugen, wenn die Auswerteeinheit (12) ein keine Wandständigkeit der Elektrodenleitung (14; 14'; 14") anzeigendes Auswertungsausgangsignal erzeugt, welches im Betrieb ein Abschalten einer Energieabgabe (24; 24'; 24") für die Ablation bewirkt.

8.  Medizinische Vorrichtung (10; 10'; 10"; 10''') nach wenigstens einem der Ansprüche 1 bis 7, wobei die Auswerteeinheit (12) ausgebildet ist, die periodischen Schwankungen eines Signalpegels des Temperatursignals (63; 63a; 63b; 63c; 63d; 63e; 63f) mit Hilfe einer Zeit-Frequenz-Transformation auszuwerten.

9.  Medizinische Vorrichtung (10; 10'; 10"; 10''') nach Anspruch 8, wobei die Auswerteeinheit (12) ausgebildet ist einen Frequenzbereich (66; 66a; 66b; 66c; 66d; 66e; 66f) des Zeit-Frequenz-transformierten Temperatursignals (65; 65a; 65b; 65c; 65d; 65e; 65f) zwischen 0,2 Hz und 4,2 Hz, bevorzugt zwischen 0,8 Hz und 3,4 Hz, insbesondere bevorzugt zwischen 1,1 Hz und 2,0 Hz auszuwerten.

10. Medizinische Vorrichtung (10; 10'; 10"; 10''') nach wenigstens einem der Ansprüche 1 bis 9, wobei die Elektrodenleitung (14; 14'; 14") biegeschlaff ist.

11. Verfahren zur Auswertung eines Temperatursignals unter Verwendung einer medizinischen Vorrichtung (10; 10'; 10"; 10''') nach wenigstens einem der Ansprüche 1 bis 10, wobei das Verfahren umfasst:

    - zur Verfügung stellen eines Temperatursignals (63; 63a; 63b; 63c; 63d; 63e; 63 f) und
    - Auswerten der periodischen Schwankungen eines Signalpegels des Temperatursignals (63; 63a; 63b; 63c; 63d; 63e; 63f), wobei ein eine Wandständigkeit der Elektrodenleitung (14; 14'; 14") qualifizierendes Auswertungsausgangsignal in Abhängigkeit davon erzeugt wird, ob periodische Schwankungen eines Signalpegels des Temperatursignals (63; 63a; 63b; 63c; 63d; 63e; 63f) unterhalb oder oberhalb eines vorbestimmten Grenzwertes liegen.

12. Verfahren nach Anspruch 11, wobei das Auswerten der periodischen Schwankungen eines Signalpegels des Temperatursignals (63; 63a; 63b; 63c; 63d; 63e; 63f) in der Auswerteeinheit (12) mit Hilfe einer Zeit-Frequenz-Transformation des Temperatursignals (63; 63a; 63b; 63c; 63d; 63e; 63f) durchgeführt wird.

13. Verfahren nach wenigstens einem der Ansprüche 11 oder 12, wobei im Mittel wenigstens 6,6 Temperaturmesswerte pro Sekunde aus dem Temperatursignal (63; 63a; 63b; 63c; 63d; 63e; 63f) ausgewertet werden.

14. Verfahren nach wenigstens einem der Ansprüche 11 bis 13 und Anspruch 5, wobei das Verfahren zusätzlich als ersten Schritt umfasst:

   - Starten einer Energieabgabe der Energiequelle (30) an die Elektrodenleitung (14; 14'; 14").

15. Verfahren nach Anspruch 14, wobei das Verfahren des Weiteren umfasst:

   - Beenden der Energieabgabe an die Elektrodenleitung (14; 14'; 14"), wenn die Auswerteeinheit (12) ein keine Wandständigkeit der Elektrodenleitung (14; 14'; 14") anzeigendes Auswertungsausgangsignal erzeugt.

FIG. 1

FIG. 2

**FIG. 3**

EP 2 856 935 A1

**FIG. 4**

EP 2 856 935 A1

**FIG. 5**

EP 2 856 935 A1

```
┌─────────────────────────┐
│          Start          │────── 52
│     Energieabgabe       │
└─────────────────────────┘
             │
             ▼
      ┌─────────────────────────┐
54 ───│          FFT            │
      │ Temperaturdaten berechnen│
      └─────────────────────────┘
                   │
                   ▼
      ┌─────────────────────────┐
56 ───│         Prüfen          │◄──────┐
      │   auf Herzfrequenzanteil │       │
      └─────────────────────────┘       │
           │              │             │
           ▼              ▼             │
   ┌──────────────┐  ┌──────────────┐   │
60 │ Energieabgabe│  │ Energieabgabe│── 58
   │ unterbrechen │  │  fortsetzen  │   │
   └──────────────┘  └──────────────┘───┘
```

**FIG. 6**

Fall 1: Idealer Wandkontakt

Kein spektraler Anteil

FIG. 7

EP 2 856 935 A1

Fall 2: Schlechter Wandkontakt

Spektraler Anteil
durch Herzfrequenz

66a

65a

1Hz          2Hz          f

f = 1,3Hz $\hat{=}$ 80 BPM          f = 2,6Hz = Harmonisch von 1,3 Hz

**FIG. 8**

FIG. 9

EP 2 856 935 A1

68c

69c

62c

63c

66c

64c

65c

**FIG. 10**

**FIG. 11**

EP 2 856 935 A1

FIG. 12

FIG. 13

Europäisches Patentamt
European Patent Office
Office européen des brevets

# EUROPÄISCHER RECHERCHENBERICHT

Nummer der Anmeldung

EP 14 18 0599

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2012/136346 A1 (CONDIE CATHERINE R [US] ET AL) 31. Mai 2012 (2012-05-31) | 1-3,6,7, 10,11, 14,15 | INV. A61B5/00 A61B5/06 A61B18/12 |
| Y | * Absätze [0052], [0054]; Anspruch 1; Abbildungen 10,11 * <br> * Absätze [0038], [0039], [0048], [0050], [0054] * <br> ----- | 8,9,12, 13 | |
| Y | US 2002/123749 A1 (JAIN MUDIT K [US]) 5. September 2002 (2002-09-05) <br> * Absätze [0013], [0075], [0076] * <br> ----- | 8,9,12, 13 | |
| A | WO 2008/035070 A2 (IMP INNOVATIONS LTD [GB]; JARMAN JULIAN WILLIAM ERNEST [GB]; FRANCIS D) 27. März 2008 (2008-03-27) <br> * Seite 25, Zeilen 13-20 * <br> ----- | 8,9,12, 13 | |

**RECHERCHIERTE SACHGEBIETE (IPC)**

A61B

Der vorliegende Recherchenbericht wurde für alle Patentansprüche erstellt

| Recherchenort | Abschlußdatum der Recherche | Prüfer |
|---|---|---|
| Den Haag | 3. März 2015 | Schmidt, Matthias |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

EP 2 856 935 A1

**ANHANG ZUM EUROPÄISCHEN RECHERCHENBERICHT
ÜBER DIE EUROPÄISCHE PATENTANMELDUNG NR.**    EP 14 18 0599

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-03-2015

| Im Recherchenbericht angeführtes Patentdokument | Datum der Veröffentlichung | Mitglied(er) der Patentfamilie | | Datum der Veröffentlichung |
|---|---|---|---|---|
| US 2012136346 A1 | 31-05-2012 | CN | 103237516 A | 07-08-2013 |
| | | EP | 2645953 A1 | 09-10-2013 |
| | | US | 2012136346 A1 | 31-05-2012 |
| | | US | 2012136348 A1 | 31-05-2012 |
| | | WO | 2012074729 A1 | 07-06-2012 |
| US 2002123749 A1 | 05-09-2002 | US | 2002123749 A1 | 05-09-2002 |
| | | US | 2002169445 A1 | 14-11-2002 |
| | | WO | 02069822 A1 | 12-09-2002 |
| WO 2008035070 A2 | 27-03-2008 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0566725 B1
- EP 2338430 B1
- EP 1827277 B1